# EUROPÄISCHE PATENTSCHRIFT

(11) **EP 4 228 787 B1**
(45) Veröffentlichungstag und Bekanntmachung des Hinweises auf die Patenterteilung: **23.04.2025**
(21) Anmeldenummer: 21805357.7
(22) Anmeldetag: 13.10.2021
(51) Int. Cl.: B01D 53/00, A61L 9/20, C02F 1/32, A61L 2/10

(54) **BESTRAHLUNGSVORRICHTUNG ZUM DEKONTAMINIEREN EINES MEDIUMS**
IRRADIATION DEVICE FOR DECONTAMINATING A MEDIUM
DISPOSITIF D'IRRADIATION POUR LA DÉCONTAMINATION D'UN MILIEU

(30) Priorität: 13.10.2020 DE 102020126849
(43) Veröffentlichungstag der Anmeldung: 23.08.2023
(73) Patentinhaber: Schweitzer Ingenieurgesellschaft Mbh, 41352 Korschenbroich (DE)
(72) Erfinder: SCHWEITZER, Bert, 41352 Korschenbroich (DE)
(74) Vertreter: Willems, Volker
(86) Internationale Anmeldenummer: PCT/DE2021/100825
(87) Internationale Veröffentlichungsnummer: WO 2022/078554

(56) Entgegenhaltungen:
- EP-A1- 1 602 628
- EP-A1- 2 100 624
- WO-A1-01/60418
- WO-A1-2017/007898
- WO-A1-2019/193825
- DE-A1- 102010 047 318
- US-A1- 2004 166 037
- US-A1- 2006 207 431
- US-A1- 2012 168 641
- US-A1- 2015 338 336
- US-A1- 2017 266 335
- US-A1- 2018 185 539

## Beschreibung

### Technisches Gebiet

Die Erfindung betrifft eine Bestrahlungsvorrichtung zum Dekontaminieren eines Mediums in einem zylinderförmigen Strömungskanal durch den das zu dekontaminierende Medium in axialer Richtung geführt wird und mit einer Innenseite, enthaltend
a) einen Strahler, welcher dekontaminierende Strahlen aussendet,
b) eine Reflektoranordnung auf der Innenseite des Strömungskanals zur Reflexion der Strahlen innerhalb des Strömungskanals.

### Beschreibung

Solche Bestrahlungsvorrichtungen zum Dekontaminieren vorzugsweise eines Fluides, bestehen im Wesentlichen aus einem zylindrischen Strahler, welcher üblicherweise von einer UV-Lampe gebildet wird, einem Strömungskanal mit oder ohne Strahlenreflektoren, sowie einem Gebläse bzw. einer Pumpe. Übliche Bauausführungen des Strömungskanals haben einen quadratischen, rechteckigen oder runden Strömungsquerschnitt. Die an den Innenwänden des Strömungskanals angebrachten UV-Reflektoren, die eine Erhöhung der Strahlungsintensität im durchströmten Raum bewirken sollen, reflektieren die emittierten Strahlen immer auch in senkrechter Richtung, so dass sie wieder auf den UV-Strahler treffen.

Die WO 2017/007898 A1 beschreibt einen photokatalytischen Reaktor mit einer Längsachse, durch die ein Fluid fließen kann. Ein Rahmen nimmt dabei eine Lichtquelle und Lamellen auf, die im Wesentlichen die Lichtquelle um die Längsachse herum umschließen. Dabei hat jede Lamelle eine innere Oberfläche, die der Lichtquelle zugewandt ist, und eine äußere Außenfläche gegenüber der Innenfläche. Mindestens ein Teil der Oberfläche von einer Vielzahl von Lamellen ist mit einem Material beschichte, welches photokatalytische und oxidative Eigenschaften aufweist. Die innere Oberfläche der Lamellen ist so ausgestaltet, dass sie jeweils einen Teil des emittierten Lichts der Lichtquelle auf einen äußeren Teil einer benachbarten Lamelle umzuleiten. In einer Ausführungsform sind die Lamellen in radialer Richtung geneigt. In einer weiteren Ausführungsform ist die Innenfläche so ausgestaltet, dass sie einen Teil des Lichts der Lichtquelle auf mindestens einen Teil der Außenfläche einer benachbarten der Außenfläche einer benachbarten Schaufel zu reflektieren. Bei diesem photokatalytischen Reaktor ist es nämlich erforderlich, dass zumindest ein Teil der Strahlen der Lichtquelle auf die Außenflächen der Lamellen umgeleitet werden, um die photokatalytischen und oxidativen Eigenschaften der Beschichtung zu aktivieren. Das Fluid muss somit den photokatalytischen Reaktor für seine Wirkweise auch außen umfließen. Von den Innenwänden der Lamellen reflektierte Strahlen, welche nicht auf die Außenseiten der Lamellen umgeleitet werden, treffen insbesondere auf die Lichtquelle und heizen diese auf. Dies reduziert die Lebensdauer der Lichtquelle in nachteiliger Weise.

Aus der US 2004/166037 A1 ist ein Luftfiltrationssystem zur Verwendung beim Filtern eines Druckluftstroms bekannt. Das System umfasst einen primären Partikelfilter zur Entfernung von mindestens einem Teil der im Luftstrom mitgeführten Partikel Luftstrom mitgerissenen Teilchen. Ferner ist mindestens eine Ultraviolettlampe und einen durchlässigen Reaktionsfilter vorgesehen. Die Ultraviolettlampe befindet sich stromabwärts in dem primären Partikelfilter und der durchlässige Reaktionsfilter ist stromabwärts des ultravioletten Lichts angeordnet. Der durchlässige Reaktionsfilter besteht aus einem Substratelement und eine Vielzahl von Titandioxidpartikeln, die an Teilen des Substratelements gebunden sind, um eine photokatalytische Oxidationsmittelschicht zu bilden. Die Ultraviolettlampe bestrahlt mindestens einen Teil der der durchlässigen Reaktionsschicht, so dass mindestens ein Teil der Verunreinigungen, die in dem Luftstrom mitgeführt werden. Das System kann auch einen Adsorptionsfilter enthalten, der stromabwärts des durchlässigen Reaktionsfilter angeordnet ist. Die reflektierten Strahlen können auf die Ultraviolettlampe treffen und diese aufheizen, wodurch die Lebensdauer dieser Lampe reduziert wird.

Bei UV-Strahlern geringer Leistung stellt dies kein Problem dar. Werden jedoch UV-Strahler mit hoher Leistung eingesetzt, führt dies zur Überhitzung des UV-Strahlers und somit zu einer geringen Lebensdauer und letztlich zu seiner Zerstörung.

### Offenbarung der Erfindung

Aufgabe der Erfindung ist es daher, die bekannten Nachteile zu vermeiden. Bestrahlungsgeräte mit Strahlern hoher Leistung könnten eine deutlich geringere Bestrahlungszeit ermöglichen, wenn auch sie in Kombination mit Reflektoren betrieben werden könnten. Es wird somit eine Lösung gesucht, die bei Verwendung von UV-Reflektoren die Beaufschlagung des UV-Strahlers mit reflektierten UV-Strahlen verhindert.

Erfindungsgemäß wird die Aufgabe dadurch gelöst, dass bei einer Bestrahlungsvorrichtung zum Dekontaminieren eines Mediums in einem Strömungskanal der eingangs genannten Art
c) die Innenwand des Strömungskanals mit Reflektoren der Reflektoranordnung ausgestattet ist, welche die reflektierten Strahlen des Strahlers an dem Strahler vorbeiführt,
d) die Reflektoranordnung die erstreflektierten Strahlen des Strahlers tangential an dem Strahler vorbeiführen und
e) der Abstand der reflektierten Strahlen zum Strahler mit der Anzahl der Reflexionen zunimmt.

Die Innenwand des Strömungskanals wird mit Reflektoren ausgestattet, die vorteilhaft segmentweise ausgeführt sind. Dabei weisen die Reflektoren Konturen auf, bei denen alle von dem Strahler radial ausgehenden Strahlen tangential an diesem vorbeigeführt werden. Dadurch wird der Strahler nicht von reflektierten Strahlen getroffen, was verhindert, dass die reflektierten Strahlen den Strahler unnötig aufheizen. Diese Überhitzung würde den Strahler ansonsten schädigen und aufgrund der Überhitzung auch zerstören. Üblicherweise handelt es sich dabei um eine Lampe, deren Lebenszeit durch diese Maßnahme nun nicht mehr beeinträchtigt wird.

Als vorteilhafte Ausgestaltung der Erfindung hat sich erwiesen, wenn bei einer erfindungsgemäßen Bestrahlungsvorrichtung der Strahler als UV-Strahler ausgebildet ist. UV-Strahlen sind für ihre dekontaminierende Wirkung, z.B. bei Bakterien, Viren oder Pilzen, bekannt. Insofern ist ein UV-Strahler für eine solche Bestrahlungsvorrichtung besonders geeignet, um Keime, Viren, Pilze und dergleichen in einem solchen Strömungskanal zu töten.

In der erfindungsgemäßen Bestrahlungsvorrichtung weist der Strahler eine zylindrische Form auf. Der Strahler wird dabei möglichst entlang der Längsachse in dem Strömungskanal positioniert. Der Strömungskanal hat dabei einen größeren Querschnitt als der Strahler, so dass zwischen Strahler und der Innenseite des Strömungskanals das zu bestrahlende Medium strömen kann. Der Strahler kann dabei entlang der gesamten Länge oder eines Längenabschnitts des Strömungskanals geführt sein. Durch die radiale Abstrahlung wird das Medium über die gesamte Länge oder einen geeigneten Längenabschnitt des Strömungskanals bestrahlt.

In einer bevorzugten Ausbildung der erfindungsgemäßen Bestrahlungsvorrichtung verstärken sich die reflektierten Strahlen durch Mehrfachreflektionen an der Reflektoranordnung. Die Reflektoranordnung besteht aus Reflektorsegmenten. Diese Reflektorsegmente sind derart ausgestaltet und auf der Innenseite des Strömungskanals angeordnet, dass sich die reflektierten Strahlen überlagern, kreuzen und somit verstärken. Damit kann ein Strahler verwendet werden, der nicht eine so hohe Leistung aufweisen muss, wie es bei der nicht verstärkenden Wirkung der Fall wäre. Außerdem kann der Strahler auf kleinstem Bauraum einen hohen Volumenstrom des Fluids dekontaminieren. Hierdurch ergibt sich zudem eine extrem kurze, erforderliche Bestrahlungszeit bei einem gegebenen Volumenstrom.

Eine vorteilhafte Ausbildung der erfindungsgemäßen Bestrahlungsvorrichtung besteht ferner darin, dass der Strahler zentrisch im Strömungskanal angeordnet ist, wobei die Strahlen radial abgestrahlt werden. Diese Maßnahme bewirkt, dass durch die radialsymmetrische Anordnung die Reflektoranordnung optimal zum Einsatz kommt. Asymmetrien haben nämlich Einfluss auf die Reflexionen und würden die Strahlungsverteilung im Strömungskanal entsprechend asymmetrisch bzw. ungleich verteilen.

Vorzugsweise strömt das Medium in axialer Richtung durch den Strömungskanal der erfindungsgemäßen Bestrahlungsvorrichtung. Diese Maßnahme erlaubt eine gleichmäßige Dekontamination des Mediums auch bei großen Volumenströmen. Das Medium muss den Strömungskanal nämlich lediglich durchströmen, um den gewünschten Dekontaminationseffekt zu erhalten. Denn der Volumenstrom wird dabei über einen längeren Weg bestrahlt.

In der erfindungsgemäßen Bestrahlungsvorrichtung besteht darin, dass der Abstand der Strahlen zum Strahler mit der Anzahl der Reflexionen zunimmt. Die dekontaminierenden Strahlen, die nach der Erstreflektion und dem Passieren des Strahlers wiederum auf die Reflektorsegmente treffen, werden prinzipbedingt mit einem etwas größeren Winkel reflektiert, so dass die Strahlen nach der zweiten Reflektion den Strahler in einem etwas größeren Abstand passieren. Dieser Effekt setzt sich mit zunehmender Reflektion fort, so dass die Strahlen nicht nur in Umfangsrichtung um den Strahler herum gelenkt werden, sondern gleichzeitig auch nach außen versetzt werden. Dies führt vorteilhaft dazu, dass im Strömungsraum einerseits eine durch die Mehrfachreflektionen bewirkte Verstärkung der Strahlungsintensität resultiert und andererseits die Strahlung sich gleichmäßig über den gesamten Strömungsquerschnitt verteilt.

Trotz der geringeren Leistung, die bei dem Strahler der erfindungsgemäßen Bestrahlungsvorrichtung erforderlich ist, heizt sich die Bestrahlungsvorrichtung insgesamt auf. Um die so entstandene Wärme abzuleiten, weist der Strömungskanal vorzugsweise Kühlelemente auf. Dies können beispielsweise einfach gestaltete Kühlrippen mit oder ohne Ventilator sein, aber auch aufwendigere Peltier-Kühlelemente oder wassergekühlte Kühlelemente, die den Wärmeüberschuss der Bestrahlungsvorrichtung abführen.

Eine weitere bevorzugte Ausgestaltung der erfindungsgemäßen Bestrahlungsvorrichtung besteht darin, dass ein Antrieb für das zu dekontaminierende Medium vorgesehen ist, welcher das Medium durch den Strömungskanal führt. Der Antrieb kann dabei beispielsweise eine Pumpe und/oder ein Gebläse sein, welches das Medium durch den Strömungskanal strömen lässt. Diese Maßnahme dient dazu, das Medium gleichmäßig und/oder die Geschwindigkeit regulierbar durch den Strömungskanal zu leiten, um immer einen gleichbleibenden Dekontaminationseffekt zu erhalten.

Weitere Ausgestaltungen und Vorteile ergeben sich aus dem Gegenstand der Unteransprüche sowie den Zeichnungen mit den dazugehörigen Beschreibungen. Ein Ausführungsbeispiel ist nachstehend unter Bezugnahme auf die beigefügten Zeichnungen näher erläutert. Die Erfindung soll nicht alleine auf dieses aufgeführte Ausführungsbeispiel beschränkt werden. Es dient lediglich zur näheren Erläuterung der Erfindung.

### Kurze Beschreibung der Zeichnung

- Fig. 1a-1c: zeigen im Querschnitt jeweils Prinzipskizzen von Bestrahlungsvorrichtungen zum Dekontaminieren von Medien, wie sie derzeit bekannt sind.
- Fig. 2: zeigt im Querschnitt einen Strömungskanal einer erfindungsgemäßen Bestrahlungsvorrichtung zum Dekontaminieren, bei der reflektierte Strahlen nicht dargestellt sind.
- Fig. 3a-3c: zeigen im Querschnitt einen Strömungskanal einer erfindungsgemäßen Bestrahlungsvorrichtung zum Dekontaminieren mit abgestrahlten und reflektierten Strahlen.
- Fig. 4: zeigt perspektivisch den Ausschnitt einer erfindungsgemäßen Bestrahlungsvorrichtung zum Dekontaminieren.
- Fig. 5: zeigt im Längsschnitt eine erfindungsgemäße Bestrahlungsvorrichtung zum Dekontaminieren mit Ventilator für das Medium.
- Fig. 6: zeigt einen perspektivischen Ausschnitt eines Strömungskanals einer erfindungsgemäßen Bestrahlungsvorrichtung mit Kühlrippen.

### Bevorzugtes Ausführungsbeispiel

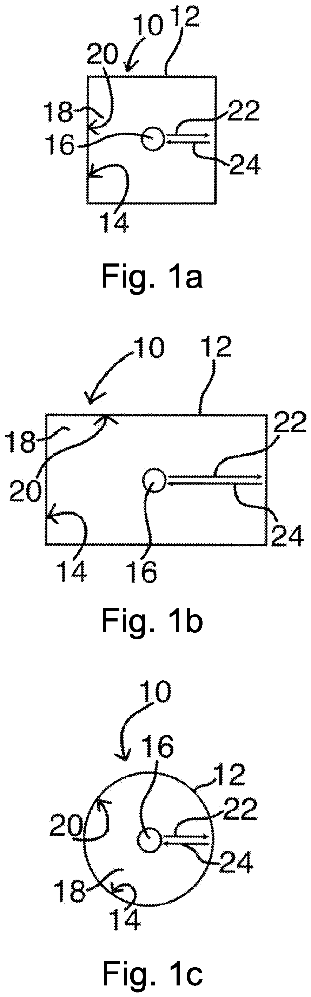

In Fig. 1a bis 1c werden drei Bauformen einer Bestrahlungsvorrichtung 10 zum Dekontaminieren eines Mediums, wie sie bekannt sind als Prinzipskizze im Querschnitt dargestellt. Dabei weist die Bestrahlungsvorrichtung 10 jeweils einen Strömungskanal 12 mit Innenseiten 14 auf, in dem ein UV-Strahler 16 zentral angeordnet ist. Der Strömungskanal 12 verfügt über einen Strömungsraum 18 und ist in Fig. 1a im Querschnitt quadratisch, in Fig. 1b rechteckig und in Fig. 1c ist der Querschnitt kreisrund. Die Figuren 1a bis 1c unterscheiden sich somit lediglich in der Form ihres Querschnitts. Die Innenseiten 14 der Strömungskanäle 12 sind jeweils als Reflektor 20 ausgebildet. Der UV-Strahler 16 besteht regelmäßig aus einer stabförmigen UV-C-Lampe. Die abgestrahlten Strahlen 22 werden von den als Reflektoren ausgebildeten Innenseiten 14 reflektiert. Dabei treffen die reflektierten Strahlen 24 wieder auf den UV-Strahler 16, welcher durch die Energie der Strahlung aufgeheizt wird.

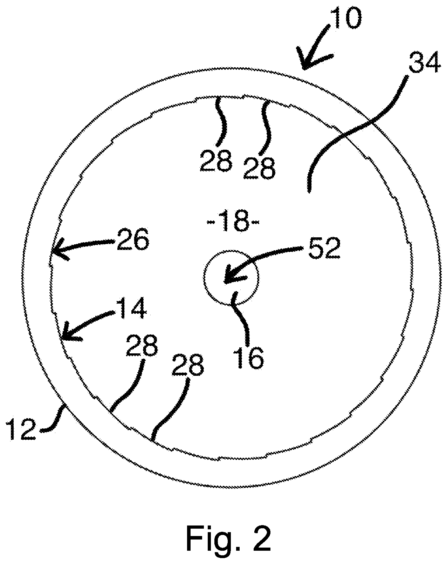

Die Fig. 2 zeigt im Querschnitt die Prinzipskizze der erfindungsgemäßen Bestrahlungsvorrichtung 10 zum Dekontaminieren eines Mediums, bei der die Strahlen 22, 24 nicht dargestellt sind. Bei dem Medium kann es sich beispielsweise um ein Fluid oder ein Gas handeln. Die Bestrahlungsvorrichtung 10 umfasst den im Querschnitt kreisrunden Strömungskanal 12. Innerhalb des Strömungskanals 12 der Bestrahlungsvorrichtung 10 befindet sich der Strömungsraum 18, durch den das zu dekontaminierende Medium 34 geführt wird.

An den Innenseiten 14 des Strömungskanals 12 der Bestrahlungsvorrichtung 10 ist eine Reflektoranordnung 26 vorgesehen, welche aus Reflektorsegmenten 28 besteht. Die Reflektoranordnung 26 besteht aus zahlreichen Reflektorsegmenten 28, welche den reflektierten Strahlen 24 aufgrund ihrer Ausrichtung und Kontur eine bestimmte Strahlenrichtung geben, welche den zentral angeordneten UV-Strahler 16 nicht treffen. Im Zentrum 52 des kreisrunden Strömungskanal 12 befindet sich der UV-Strahler 16 als Strahlenquelle. Die abgestrahlten Strahlen 22 des UV-Strahlers 16 werden radial abgestrahlt und treffen auf die Reflektorsegmente 28 der Reflektoranordnung 26.

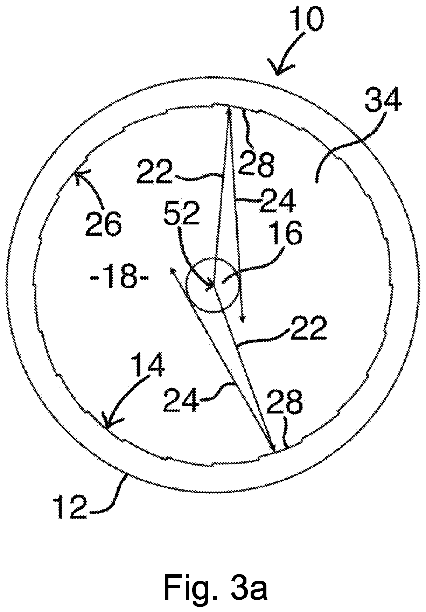

Die Reflektorsegmente 28 der Reflektoranordnung 26 sind derart ausgerichtet, dass die reflektierten Strahlen 24 den UV-Strahler 16 nicht mehr beaufschlagen, d. h. tangential vorbeistrahlen (siehe Fig. 3a-3c). Die Reflektoranordnung 26 ist zudem so ausgestaltet, dass bei jeder Reflexion eines reflektierten Strahls 24 der radiale Abstand zur Strahlenquelle 16 größer wird.

Alle radial emittierten Strahlen 22 des zentrisch im Strömungskanal 12 platzierten UV-Strahlers lenken bei der Erstreflektion unter einem gleichen Winkel so um, dass der UV-Strahler 16 nicht beaufschlagt wird. Die anschließenden weiteren Reflektionen der umlaufenden UV-Strahlen 24 führen zu einem radialen Versatz der UV-Strahlen 24 nach außen. Durch eine zusätzliche Neigung des Strömungskanals 12 in Strömungsrichtung können die UV-Strahlen gleichzeitig axial in Strömungsrichtung umgelenkt und bei Verwendung eines entgegengesetzt geneigten Strömungskanal 12 wieder zurückgeführt werden.

Fig. 3a-3c zeigen im Querschnitt den Strömungskanal 12 der erfindungsgemäßen Bestrahlungsvorrichtung 10 zum Dekontaminieren mit abgestrahlten und reflektierten Strahlen 22, 24. Dabei werden in Fig. 3a von dem UV-Strahler 16 beispielhaft zwei Strahlen 22 radial abgestrahlt, welche an zwei Reflektorsegmenten 28 der Reflektoranordnung 26 einmal reflektiert werden. Die Reflektorsegmente 28 sind so ausgerichtet und so geformt, dass die reflektierenden Strahlen 24 den UV-Strahler 16 nicht mehr treffen können und tangential an ihm vorbeigelenkt werden.

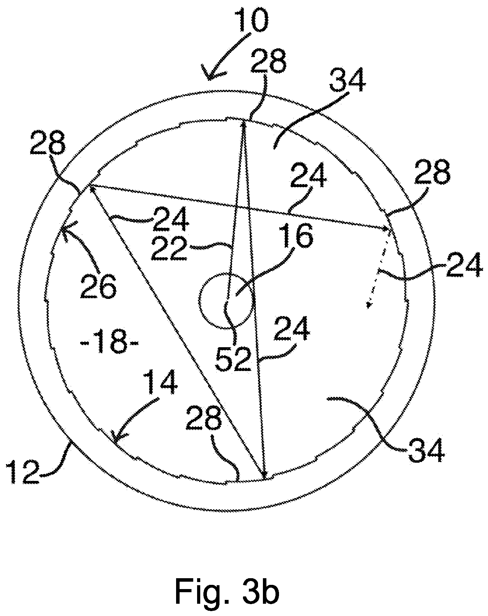

Die Fig. 3b zeigt beispielhaft, wie ein einzelner aus dem UV-Strahler 16 austretender Strahl 22 mehrfach reflektiert wird. Nach der ersten Reflexion des Strahls 22 liegt der reflektierte Strahl 24 noch sehr dicht beim Zentrum 52, der Strahlenquelle 16. Mit zunehmender Zahl der Reflexionen wächst jedoch auch der Abstand zum Zentrum 52.

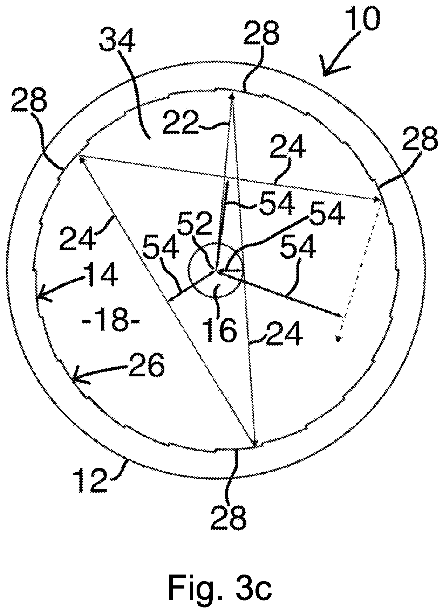

Fig. 3c zeigt beispielhaft, wie ein einzelner aus dem UV-Strahler 16 austretender Strahl 22 mehrfach reflektiert wird gemäß Fig. 3b. Allerdings sind hier noch Abstände 54 der einzelnen reflektierten Strahlen 24 vom Zentrum 52 zusätzlich eingezeichnet.

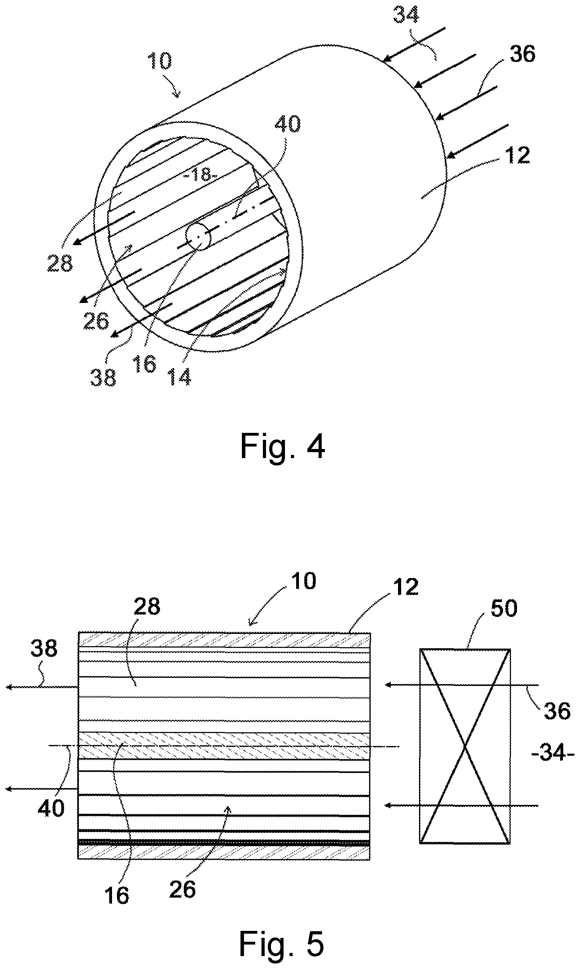

Die Fig. 4 zeigt perspektivisch die Prinzipskizze der erfindungsgemäßen Bestrahlungsvorrichtung 10 zum Dekontaminieren. In den Strömungskanal 12 der Bestrahlungsvorrichtung 10 strömt ein zu dekontaminierendes Medium 34 ein. Das in den Strömungsraum 18 einströmende Medium wird mit Pfeilen 36 und das ausströmende Medium 34 mit Pfeilen 38 symbolisiert. Der stabförmige UV-Strahler 16 sitzt auf einer Längsachse 40 zentral in dem Strömungskanal 12.

Fig. 5 zeigt im Längsschnitt die Prinzipskizze der erfindungsgemäßen Bestrahlungsvorrichtung 10 zum Dekontaminieren eines Mediums 34 entsprechend der Fig. 4. Im Gegensatz zu Fig. 4 ist ein Ventilator 50 als Gebläse für das Medium 34 vorgesehen. Außerdem ist die Reflektoranordnung 26 so ausgebildet, dass sie rotiert. Wahlweise kann die Reflektoranordnung 26 innerhalb des Strömungskanals 12 oder mit ihm rotieren. Durch Überlagerung der Strahlen entstehen im Strömungsraum 18 Bereiche mit höherer und geringerer Strahlenintensität. Durch die Rotation der Reflektoranordnung 26 wird dieser Effekt gemildert. Die Rotation bewirkt eine gleichmäßigere Strahlungsintensität in dem Strömungsraum 18. Außerdem wird durch die Rotation die Bestrahlungsvorrichtung 10 gekühlt. Der Ventilator 50 dient als Antrieb für das Medium 34, um es in den Strömungskanal 12 der Bestrahlungsvorrichtung 10 zu leiten. Die Strömungsgeschwindigkeit des Mediums 34 kann dabei über den Ventilator 50 reguliert werden. Ein langsam strömendes Medium 34 hat eine längere Verweilzeit im Strömungsraum 18 und kann länger bestrahlt bzw. dekontaminiert werden. Entsprechend verkürzt sich die Verweilzeit bei einer höheren Strömungsgeschwindigkeit des Mediums 34.

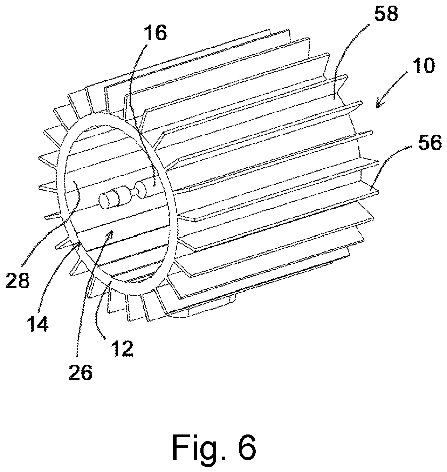

Fig. 6 zeigt einen dreidimensionalen Ausschnitt eines Strömungskanals 12 einer erfindungsgemäßen Bestrahlungsvorrichtung 10 mit Kühlrippen 56. In dem zylinderförmigen Strömungskanal 12 ist zentral der UV-Strahler 16 angeordnet. Der UV-Strahler 16 ist eine längliche und ebenfalls zylinderförmige Lampe, die axial angeordnet ist. Die Reflektoranordnung 26 ist auf der Innenseite 14 des Strömungskanals 12 angeordnet. Auf der Außenseite 58 des Strömungskanals 12 sind die Kühlrippen 56 der Bestrahlungsvorrichtung 10 befestigt. Die Kühlrippen 56 geben Wärme der Bestrahlungsvorrichtung 10 durch ihre große Oberfläche ab. Bei einem rotierenden Strömungskanal 12, wie es zu Fig. 5 beschrieben wurde, wird die Kühlwirkung durch die Kühlrippen 56 zusätzlich verstärkt.

### Bezugszeichenliste

- 10: Bestrahlungsvorrichtung
- 12: Strömungskanal
- 14: Innenseiten
- 16: UV-Strahler
- 18: Strömungsraum
- 20: Reflektor
- 22: Abgestrahlte Strahlen
- 24: Reflektierte Strahlen
- 26: Reflektoranordnung
- 28: Reflektorsegmente
- 34: Medium
- 36: Pfeile
- 38: Pfeile
- 40: Längsachse
- 50: Ventilator
- 52: Zentrum
- 54: Abstände
- 56: Kühlrippen
- 58: Außenseite

## Patentansprüche

1. Bestrahlungsvorrichtung (10) zum Dekontaminieren eines Mediums (34) in einem zylinderförmigen Strömungskanal (12) mit einer Innenseite (14) durch den das zu dekontaminierende Medium (34) in axialer Richtung geführt wird, enthaltend
a) einen Strahler (16), welcher dekontaminierende Strahlen (22) aussendet,
b) eine Reflektoranordnung (26) auf der Innenseite (14) des Strömungskanals (12) zur Reflexion der Strahlen (24) innerhalb des Strömungskanals (12), wobei
c) die Innenwand des Strömungskanals mit Reflektoren der Reflektoranordnung (26) ausgestattet ist, welche die reflektierten Strahlen (24) des Strahlers (16) an dem Strahler (16) vorbeiführt, wobei
d) die Reflektoranordnung (26) die erstreflektierten Strahlen (24) des Strahlers (16) tangential an dem Strahler (16) vorbeiführen und
e) der Abstand (54) der reflektierten Strahlen (24) zum Strahler (16) mit der Anzahl der Reflexionen zunimmt.

2. Bestrahlungsvorrichtung (10) zum Dekontaminieren eines Mediums (34) in einem Strömungskanal (12) nach Anspruch 1, **dadurch gekennzeichnet, dass** die Reflektoranordnung (26) Reflektorsegmente (28) enthält, die auf der Innenseite (14) des Strömungskanals (12) angeordnet sind.

3. Bestrahlungsvorrichtung (10) zum Dekontaminieren eines Mediums (34) in einem Strömungskanal (12) nach einem der Ansprüche 1 oder 2, **dadurch gekennzeichnet, dass** der Strahler als UV-Strahler (16) ausgebildet ist.

4. Bestrahlungsvorrichtung (10) zum Dekontaminieren eines Mediums (34) in einem Strömungskanal (12) nach einem der Ansprüche 2 oder 3, wenn dieser vom Anspruch 2 abhängig ist, **dadurch gekennzeichnet, dass** die reflektierten Strahlen (24) sich durch Mehrfachreflektionen an den Reflektorsegmenten (28) der Reflektoranordnung (26) auf der Innenseite (14) des Strömungskanals (12) durch Überlagern oder Kreuzen verstärken.

5. Bestrahlungsvorrichtung (10) zum Dekontaminieren eines Mediums (34) in einem Strömungskanal (12) nach Anspruch 4 , **dadurch gekennzeichnet, dass** der Strahler (16) zentrisch im Strömungskanal (12) angeordnet ist, wobei die Strahlen (22) radial abgestrahlt werden.

6. Bestrahlungsvorrichtung (10) zum Dekontaminieren eines Mediums (34) in einem Strömungskanal (12) nach einem der Ansprüche 1 bis 5, **dadurch gekennzeichnet, dass** der Strömungskanal (12) Kühlelemente (56) aufweist.

7. Bestrahlungsvorrichtung (10) zum Dekontaminieren eines Mediums (34) in einem Strömungskanal (12) nach einem der Ansprüche 1 bis 6, **dadurch gekennzeichnet, dass** ein Antrieb (50) für das zu dekontaminierende Medium (34) vorgesehen ist, welcher das Medium (34) durch den Strömungskanal (12) leitet.

## Claims

1. Irradiation device (10) for decontaminating a medium (34) in a cylindrical flow channel (12) with an inner surface (14), through which the medium (34) to be decontaminated is conducted in an axial direction, comprising:
a) an emitter (16) that emits decontaminating radiation (22);
b) a reflector arrangement (26) positioned on the inner surface (14) of the flow channel (12) to reflect radiation (24) within the flow channel (12); wherein
c) the inner wall of the flow channel is equipped with reflectors from the reflector arrangement (26), which direct the reflected radiation (24) from the emitter (16) past the emitter (16);
d) the reflector arrangement (26) guides the initially reflected radiation (24) of the emitter (16) tangentially past the emitter (16); and
e) the distance (54) of the reflected radiation (24) from the emitter (16) increases with the number of reflections.

2. Irradiation device (10) for decontaminating a medium (34) in a flow channel (12) according to claim 1, **characterized in that** the reflector arrangement (26) comprises reflector segments (28) arranged on the inner surface (14) of the flow channel (12).

3. Irradiation device (10) for decontaminating a medium (34) in a flow channel (12) according to claim 1 or 2, **characterized in that** the emitter is configured as a UV emitter (16).

4. Irradiation device (10) for decontaminating a medium (34) in a flow channel (12) according to claim 2 or 3, when dependent on claim 2, **characterized in that** the reflected radiation (24) is intensified through multiple reflections at the reflector segments (28) of the reflector arrangement (26) on the inner surface (14) of the flow channel (12) via superposition or crossing.

5. Irradiation device (10) for decontaminating a medium (34) in a flow channel (12) according to claim 4, **characterized in that** the emitter (16) is centrally arranged in the flow channel (12), with radiation (22) being emitted radially.

6. Irradiation device (10) for decontaminating a medium (34) in a flow channel (12) according to any of claims 1 to 5, **characterized in that** the flow channel (12) includes cooling elements (56).

7. Irradiation device (10) for decontaminating a medium (34) in a flow channel (12) according to any of claims 1 to 6, **characterized in that** a drive (50) is provided for conveying the medium (34) through the flow channel (12).

## Revendications

1. Dispositif d'irradiation (10) pour la décontamination d'un milieu (34) dans un canal d'écoulement cylindrique (12) avec une surface interne (14), à travers lequel le milieu (34) à décontaminer est acheminé dans une direction axiale, comprenant:
a) un émetteur (16) émettant un rayonnement de décontamination (22) ;
b) un agencement de réflecteurs (26) positionné sur la surface interne (14) du canal d'écoulement (12) pour réfléchir le rayonnement (24) à l'intérieur du canal d'écoulement (12) ; où
c) la paroi interne du canal d'écoulement est équipée de réflecteurs de l'agencement de réflecteurs (26), qui dirigent le rayonnement réfléchi (24) de l'émetteur (16) au-delà de l'émetteur (16) ;
d) l'agencement de réflecteurs (26) guide le rayonnement réfléchi initial (24) de l'émetteur (16) tangentiellement au-delà de l'émetteur (16) ; et
e) la distance (54) du rayonnement réfléchi (24) par rapport à l'émetteur (16) augmente avec le nombre de réflexions.

2. Dispositif d'irradiation (10) pour la décontamination d'un milieu (34) dans un canal d'écoulement (12) selon la revendication 1, **caractérisé en ce que** l'agencement de réflecteurs (26) comprend des segments de réflecteurs (28) disposés sur la surface interne (14) du canal d'écoulement (12).

3. Dispositif d'irradiation (10) pour la décontamination d'un milieu (34) dans un canal d'écoulement (12) selon la revendication 1 ou 2, **caractérisé en ce que** l'émetteur est configuré comme un émetteur UV (16).

4. Dispositif d'irradiation (10) pour la décontamination d'un milieu (34) dans un canal d'écoulement (12) selon la revendication 2 ou 3, lorsque dépendant de la revendication 2, **caractérisé en ce que** le rayonnement réfléchi (24) est intensifié par des réflexions multiples sur les segments de réflecteurs (28) de l'agencement de réflecteurs (26) sur la surface interne (14) du canal d'écoulement (12) via superposition ou croisement.

5. Dispositif d'irradiation (10) pour la décontamination d'un milieu (34) dans un canal d'écoulement (12) selon la revendication 4, **caractérisé en ce que** l'émetteur (16) est disposé de manière centrale dans le canal d'écoulement (12), le rayonnement (22) étant émis radialement.

6. Dispositif d'irradiation (10) pour la décontamination d'un milieu (34) dans un canal d'écoulement (12) selon l'une des revendications 1 à 5, **caractérisé en ce que** le canal d'écoulement (12) comprend des éléments de refroidissement (56).

7. Dispositif d'irradiation (10) pour la décontamination d'un milieu (34) dans un canal d'écoulement (12) selon l'une des revendications 1 à 6, **caractérisé en ce qu'un** entraînement (50) est prévu pour transporter le milieu (34) à travers le canal d'écoulement (12).
